(19) Europäisches Patentamt
European Patent Office
Office européen des brevets

(11) EP 3 858 324 A1

(12) **EUROPEAN PATENT APPLICATION**
published in accordance with Art. 153(4) EPC

(43) Date of publication:
**04.08.2021 Bulletin 2021/31**

(21) Application number: **19864331.4**

(22) Date of filing: **30.09.2019**

(51) Int Cl.:
***A61K 8/34*** *(2006.01)* ***A61K 8/36*** *(2006.01)*
***A61K 8/37*** *(2006.01)* ***A61K 8/81*** *(2006.01)*
***A61K 8/86*** *(2006.01)* ***A61K 8/891*** *(2006.01)*
***A61Q 1/00*** *(2006.01)*

(86) International application number:
**PCT/JP2019/038448**

(87) International publication number:
**WO 2020/067560 (02.04.2020 Gazette 2020/14)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **28.09.2018 JP 2018185465**

(71) Applicant: **Kao Corporation**
**Chuo-ku,**
**Tokyo 103-8210 (JP)**

(72) Inventors:
- **HIRONO, Shingo**
  **Tokyo 131-8501 (JP)**
- **OHBA, Chihiro**
  **Wakayama-shi, Wakayama 640-8580 (JP)**
- **ISEKI, Tomokazu**
  **Wakayama-shi, Wakayama 640-8580 (JP)**

(74) Representative: **Hoffmann Eitle**
**Patent- und Rechtsanwälte PartmbB**
**Arabellastraße 30**
**81925 München (DE)**

# (54) EXTERNAL PREPARATION

(57) An external preparation containing the following component (A) and component (B). Component (A): ionic polymer particles containing structural units derived from (a) one or more hydrophobic monomers selected from the group consisting of styrene and a derivative thereof, a vinyl ester, and a hydrophobic acrylic monomer, and (b) an ionic hydrophilic monomer or a salt thereof, wherein the ratio by mass of (a) to (b), (a)/(b) is 99.5/0.5 to 80/20, and the ionic polymer particles have a glass transition temperature of higher than 5°C and 120°C or lower. Component (B): one or more compounds selected from the group consisting of a phenoxyethanol (B1), a 2-ethylhexyl paramethoxycinnamate (B2), a benzyl alcohol (B3), a sorbitol (B4), a specific oxyalkylene derivative (B5), a polyoxyalkylene alkyl glucoside (B6), an oleic acid (B7), an alkyl benzoate (B8), a xylitol (B9), a 2-ethylhexyl (RS)-2-cyano-3,3-diphenylprop-2-enoate (B10), and a methylphenylpolysiloxane (B11).

EP 3 858 324 A1

## Description

Field of the Invention

The present invention relates to an external preparation.

Background of the Invention

In external preparations such as skin cosmetics, use of an acrylic polymer emulsion for enhancing waterproofness, sebum resistance and adhesiveness to skin is known.
For example, PTL 1 discloses that an aqueous polymer emulsion prepared by emulsion polymerization of a specific monomer has good storage stability and a coating film thereof is excellent in waterproofness and adhesiveness.
PTL 2 discloses that a cosmetic material resin prepared by emulsion polymerization of a specific monomer mixture is excellent in transparency and is excellent in the balance of characteristics such as waterproofness, adhesiveness, makeup lasting performance and skin tightness.
PTL 3 discloses that a water-in-oil skin cosmetic material containing a specific resin emulsion, a partially-crosslinked organopolysiloxane polymer, and a volatile silicone but substantially not containing a surfactant is excellent in transparency, waterproofness and sebum resistance, has a lubricious feeling and has good storage stability.
PTL 4 discloses that a soap-free polymer emulsion for cosmetics, which is produced by copolymerizing a specific hydrophobic monomer and a specific hydrophilic monomer and has a glass transition temperature of 5 to -50°C forms a soft film and is excellent in formulation stability (ethanol resistance) and applicability (wrinkling resistance).

Citation List

Patent Literature

PTL 1: JP 2002-327019 A
PTL 2: JP 2005-2207 A
PTL 3: JP 2006-8585 A
PTL 4: JP 2006-8561 A

Summary of the Invention

The present invention relates to an external preparation containing the following component (A) and component (B):

Component (A):

ionic polymer particles containing structural units derived from:

(a) one or more hydrophobic monomers selected from the group consisting of styrene and a derivative thereof, a vinyl ester, and a hydrophobic acrylic monomer, and
(b) an ionic hydrophilic monomer or a salt thereof, wherein:

the ratio by mass of (a) to (b), (a)/(b) is 99.5/0.5 to 80/20, and the ionic polymer particles have a glass transition temperature of higher than 5°C and 120°C or lower, and

Component (B):
one or more compounds selected from the group consisting of a phenoxyethanol (B1), a 2-ethylhexyl paramethoxycinnamate (B2), a benzyl alcohol (B3), a sorbitol (B4), an oxyalkylene derivative (B5) represented by the following general formula (I), a polyoxyalkylene alkyl glucoside (B6), an oleic acid (B7), an alkyl benzoate (B8), a xylitol (B9), a 2-ethylhexyl (RS)-2-cyano-3,3-diphenylprop-2-enoate (B10), and a methylphenylpolysiloxane (B11):

$$Z\text{-}\{O(PO)_1(EO)_m\text{-}(BO)_nH\}_a \qquad (I)$$

wherein Z represents a residue of a compound containing 3 or more and 9 or less hydroxy groups, as derived by

removing a's hydroxy groups from the compound, PO represents an oxypropylene group, EO represents an oxyethylene group, BO represents an oxyalkylene group having 4 carbon atoms, a falls within a range of 3 or more and 9 or less, 1, m and n each represent an average addition molar number of PO, EO and BO units, respectively, 1 is 0.5 or more and 10 or less, m is 1 or more and 20 or less, n is 0.5 or more and 5 or less, provided that the ratio by mass (PO/EO) falls within a range of 1/5 to 5/1.

Detailed Description of the Invention

[External Preparation]

The external preparation of the present invention contains the following component (A) and component (B):

Component (A):

ionic polymer particles containing structural units derived from:

(a) one or more hydrophobic monomers selected from the group consisting of styrene and a derivative thereof, a vinyl ester, and a hydrophobic acrylic monomer, and
(b) an ionic hydrophilic monomer or a salt thereof, wherein:

the ratio by mass of (a) to (b), (a)/(b) is 99.5/0.5 to 80/20, and the ionic polymer particles have a glass transition temperature of higher than 5°C and 120°C or lower, and

Component (B):
one or more compounds selected from the group consisting of a phenoxyethanol (B1), a 2-ethylhexyl paramethoxycinnamate (B2), a benzyl alcohol (B3), a sorbitol (B4), an oxyalkylene derivative (B5) represented by the following general formula (I), a polyoxyalkylene alkyl glucoside (B6), an oleic acid (B7), an alkyl benzoate (B8), a xylitol (B9), a 2-ethylhexyl (RS)-2-cyano-3,3-diphenylprop-2-enoate (B10), and a methylphenylpolysiloxane (B11):

$$Z\text{-}\{O(PO)_1(EO)_m\text{-}(BO)_nH\}_a \qquad (I)$$

wherein Z represents a residue of a compound containing 3 or more and 9 or less hydroxy groups, as derived by removing a's hydroxy groups from the compound, PO represents an oxypropylene group, EO represents an oxyethylene group, BO represents an oxyalkylene group having 4 carbon atoms, a falls within a range of 3 or more and 9 or less, 1, m and n each represent an average addition molar number of PO, EO and BO units, respectively, 1 is 0.5 or more and 10 or less, m is 1 or more and 20 or less, n is 0.5 or more and 5 or less, provided that the ratio by mass (PO/EO) falls within a range of 1/5 to 5/1.

Containing the above-mentioned component (A) and component (B), the external preparation of the present invention is less sticky and excellent in good feeling in use, and in addition, has a high-level film forming performance and is excellent in wear resistance.

In conventional technologies, an acrylic polymer emulsion or an external preparation containing it still has room for improvement in point of satisfying both less stickiness and good feeling in use, and excellent film forming performance and good wear resistance of the formed film.

An object of the present invention is to provide an external preparation that is less sticky and excellent in good feeling in use, and in addition, has a high-level film forming performance and is excellent in wear resistance.

The present inventors have found that an external preparation containing a combination of predetermined ionic polymer particles and a predetermined compound can solve the above-mentioned problems.

The external preparation of the present invention is less sticky and excellent in good feeling in use, and in addition, has a high-level film forming performance and is excellent in wear resistance, and is therefore useful for, for example, skin cosmetics.

The reason why the external preparation of the present invention provides the above-mentioned advantageous effects can be considered to be as follows.

By emulsifying an ionic polymer in water, the resultant ionic polymer particles can be used in various external preparations such as skin cosmetics. However, when the glass transition temperature (Tg) of the polymer used is low, the resultant emulsion is extremely sticky and the feeling thereof in use is poor. On the other hand, when Tg of the polymer is too high, the film forming performance thereof lowers and the wear resistance and the waterproofness of the formed film tend to lower.

In the external preparation of the present invention, ionic polymer particles having Tg of higher than 5°C are used as the component (A), and can therefore suppress stickiness and improve feeling in use. In addition, the component (A) has a structural unit derived from a specific hydrophobic monomer, and therefore has a good affinity with the component (B) to be mentioned hereinunder, and consequently, it is considered that, by using the component (A) and the component (B) as combined, the film forming performance of the resultant external preparation can be high and the wear resistance and the waterproofness of the film to be formed can be good, even though Tg of the component (A) is higher than 5°C and falls within a relatively high range. Further, when Tg of the component (A) is 120°C or lower, the resultant external preparation can keep a good film forming performance.

The external preparation of the present invention can be, for example, an external preparation to be applied to hair and skin. From the viewpoint of the advantageous effects thereof that the external preparation of the present invention is less sticky and excellent in good feeling in use and that the film formed of it has good wear resistance, the external preparation of the present invention is preferably a cosmetic material, more preferably a skin cosmetic material.

The formulation of the external preparation is not specifically limited, and any type of formulations is employable, including liquid, foam, paste, cream, and solid.

Preferably, the external preparation of the present invention is an oil-in-water type external preparation in which the component (A) and the component (B) form an oil-in-water emulsion.

<Component (A): ionic polymer particles>

The external preparation of the present invention contains, as the component (A):

ionic polymer particles containing structural units derived from:

(a) one or more hydrophobic monomers selected from the group consisting of styrene and a derivative thereof, a vinyl ester, and a hydrophobic acrylic monomer, and
(b) an ionic hydrophilic monomer or a salt thereof, wherein:

the ratio by mass of (a) to (b), (a)/(b) is 99.5/0.5 to 80/20, and the ionic polymer particles have a glass transition temperature of higher than 5°C and 120°C or lower.

Containing the component (A) and owing to the advantageous effects thereof mentioned above, the external preparation of the present invention is less sticky and excellent in good feeling in use, and in addition, the film formed of it has good wear resistance.

"Hydrophobic monomer" in this description means a monomer such that the homopolymer thereof has a solubility in water at 20°C of 1% by mass or less, and "hydrophilic monomer" means a monomer such that the homopolymer thereof has a solubility in water at 20°C of more than 1% by mass. Specific examples of the hydrophobic monomer and the hydrophilic monomer are described below.

(Glass Transition Temperature (Tg))

The ionic polymer particles of the component (A) have a glass transition temperature (Tg) of higher than 5°C and 120°C or lower. Since Tg is higher than 5°C, the external preparation can suppress stickiness and can improve the feeling thereof in use, and since Tg is 120°C or lower, the external preparation can secure a good film forming performance.

From the viewpoint of suppressing stickiness, Tg of the ionic polymer particles is preferably 10°C or higher, more preferably 15°C or higher, even more preferably 20°C or higher, further more preferably 30°C or higher, and from the viewpoint of improving the film forming performance, that is, improving the wear resistance of the formed film, Tg is preferably 105°C or lower, more preferably 100°C or lower, even more preferably 95°C or lower, further more preferably 70°C or lower, further more preferably 50°C or lower, further more preferably 45°C or lower.

A specific range of Tg of the ionic polymer particles is preferably 10 to 105°C, more preferably 10 to 100°C, even more preferably 15 to 100°C, further more preferably 20 to 100°C, further more preferably 30 to 95°C, further more preferably 30 to 70°C, further more preferably 30 to 50°C, further more preferably 30 to 45°C.

The glass transition temperature of the ionic polymer particles can be measured with an ordinary differential scanning calorimeter (DSC) using fully dried polymer particles. Or in the case where Tg of the homopolymer of each monomer that constitutes the polymer particles is known, the glass transition temperature can be calculated according to the following mathematical expression.

$$\frac{1}{273+Tg}=\frac{w_1}{273+Tg_1}+\frac{w_2}{273+Tg_2}+\cdot\cdot\cdot \quad (1)$$

wherein $w_1$, $w_2$, ... each are a weight fraction of each monomer, and the total of the weight fractions is 1; $Tg_1$, $Tg_2$, ... each are Tg (°C) of the homopolymer of each monomer.

The glass transition temperature of the homopolymer of each monomer is described, for example, in J. Brandrup, et. al., "Polymer Handbook, Fourth Edition", John Wiley & Sons. Inc.

(Hydrophobic Monomer (a))

The component (A) has a structural unit derived from (a) one or more hydrophobic monomers selected from the group consisting of styrene and a derivative thereof, a vinyl ester, and a hydrophobic acrylic monomer (hereinafter may be simply referred to as "hydrophobic monomer (a)").

[Styrene and Derivative thereof]

The styrene and derivatives thereof usable as the hydrophobic monomer (a) include styrene, α-methylstyrene, methylstyrene, butylstyrene, t-butylstyrene, dimethylstyrene, and divinylbenzene. One or more of these can be used. Among these, from the viewpoint of easiness in emulsion polymerization in production of polymer particles, and from availability and economic potential, styrene is preferred.

[Vinyl Ester]

The vinyl ester usable as the hydrophobic monomer (a) includes vinyl esters having an alkyl group or an alkenyl group, such as vinyl acetate, vinyl propionate, vinyl butyrate, vinyl hexanoate, vinyl octanoate, vinyl decanoate, vinyl laurate, vinyl palmitate, and vinyl stearate, and one or more of these can be used. Among these, from the viewpoint of easiness in emulsion polymerization in production of polymer particles, and from availability and economic potential, vinyl acetate is preferred.

[Hydrophobic Acrylic Monomer]

The hydrophobic acrylic monomer usable as the hydrophobic monomer (a) is preferably (meth)acrylate, and is, for example, a (meth)acrylate represented by the following general formula (1), and the homopolymer thereof has a solubility in water at 20°C of 1% by mass or less. In this description, "(meth)acrylic acid" means methacrylic acid or acrylic acid.

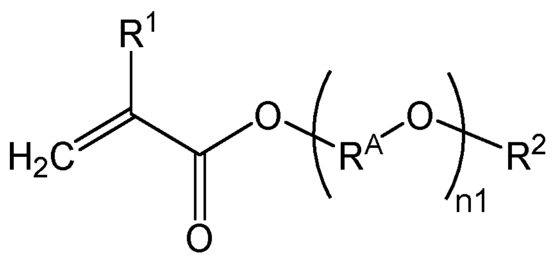

(1)

In the formula (1), $R^1$ represents a hydrogen atom or a methyl group, $R^2$ represents a chainlike aliphatic group having 1 or more and 24 or less carbon atoms, a cycloaliphatic group having 5 or more and 24 or less carbon atoms, an aryl group having 6 or more and 24 or less carbon atoms, or an aralkyl group having 7 or more and 24 or less carbon atoms, which may optionally have a hydroxy group, $R^A$ represents an alkylene group having 2 or more and 4 or less carbon atoms. n1 represents an integer of 0 or more and 30 or less carbon atoms.

In the formula (1), $R^1$ represents a hydrogen atom or a methyl group, $R^2$ represents a chainlike aliphatic group having 1 or more and 24 or less carbon atoms, a cycloaliphatic group having 5 or more and 24 or less carbon atoms, an aryl group having 6 or more and 24 or less carbon atoms, or an aralkyl group having 7 or more and 24 or less carbon atoms, which may optionally have a hydroxy group. The chainlike aliphatic group may be any of a straight chainlike aliphatic group or a branched chainlike aliphatic group.

$R^2$ is, from the viewpoint of stickiness suppression, easiness in emulsion polymerization to produce polymer particles, and availability and economic potential, preferably a chainlike aliphatic group having 1 or more and 24 or less carbon atoms, more preferably an alkyl group having 1 or more and 24 or less carbon atoms, even more preferably an alkyl group having 1 or more and 12 or less carbon atoms, further more preferably an alkyl group having 1 or more and 8 or less carbon atoms, further more preferably an alkyl group having 1 or more and 6 or less carbon atoms.

$R^A$ represents an alkylene group having 2 or more and 4 or less carbon atoms, and is, from the viewpoint of availability and economic potential, preferably an ethylene group or a propylene group. When n1 is 2 or more, plural $R^A$'s may be the same as or different from each other.

n1 is preferably an integer of 0 or more and 10 or less, and is, from the viewpoint of stickiness suppression, easiness in emulsion polymerization to produce polymer particles, and availability and economic potential, more preferably 0.

Among the (meth)acrylate represented by the general formula (1) for the hydrophobic acrylic monomer, an alkyl (meth)acrylate is preferred from the viewpoint of easiness in emulsion polymerization in production of polymer particles, and from availability and economic potential. The carbon number of alkyl is preferably 1 or more and 24 or less, more preferably 1 or more and 12 or less, even more preferably 1 or more and 8 or less, further more preferably 1 or more and 6 or less.

Specific examples of the alkyl (meth)acrylate include one or more selected from the group consisting of methyl (meth)acrylate, ethyl (meth)acrylate, n-propyl (meth)acrylate, isopropyl (meth)acrylate, n-butyl (meth)acrylate, isobutyl (meth)acrylate, sec-butyl (meth)acrylate, tert-butyl (meth)acrylate, 2-ethylhexyl (meth)acrylate, isooctyl (meth)acrylate, n-decyl (meth)acrylate, isodecyl (meth)acrylate and lauryl (meth)acrylate.

The hydrophobic monomer (a) is, from the viewpoint of improving wear resistance of film, and preventing stickiness thereof, and from the viewpoint of easiness in emulsion polymerization to produce polymer particles, and availability and economic potential, preferably one or more selected from the group consisting of styrene, vinyl acetate and an alkyl (meth)acrylate, more preferably one or more selected from the group consisting of styrene and an alkyl (meth)acrylate, even more preferably one or more selected from the group consisting of styrene and an alkyl (meth)acrylate in which the alkyl moiety has 1 or more and 8 or less carbon atoms, further more preferably one or more selected from the group consisting of styrene and an alkyl (meth)acrylate in which the alkyl moiety has 1 or more and 6 or less carbon atoms.

From the viewpoint of controlling Tg of the ionic polymer particles of the component (A), the hydrophobic monomer (a) preferably contains the following monomer (a1) and monomer (a2).

(a1) One or more selected from the group consisting of styrene, vinyl acetate, and a (meth)acrylate such that the homopolymer thereof has a glass transition temperature of higher than 5°C.
(a2) A (meth)acrylate such that the homopolymer thereof has a glass transition temperature of 5°C or lower.

<<Monomer (a1)>>

The monomer (a1) is one or more selected from the group consisting of styrene, vinyl acetate, and a (meth)acrylate such that the homopolymer thereof has a glass transition temperature of higher than 5°C.

Among the monomer (a1), the (meth)acrylate such that the homopolymer thereof has a glass transition temperature of higher than 5°C is preferably a (meth)acrylate of the general formula (1) such that the homopolymer thereof has a glass transition temperature of higher than 5°C.

From the viewpoint of easiness in emulsion polymerization to produce polymer particles and availability and economic potential, the monomer (a1) is preferably one or more selected from the group consisting of styrene and an alkyl (meth)acrylate in which the carbon number of the alkyl moiety is preferably 1 or more and 24 or less, more preferably 1 or more and 12 or less, even more preferably 1 or more and 8 or less, further more preferably 1 or more and 6 or less.

Specific examples of the monomer (a1) include styrene (100°C), vinyl acetate (32°C), methyl acrylate (10°C), tert-butyl acrylate (107°C), isobornyl acrylate (94°C), methyl methacrylate (105°C), ethyl methacrylate (65°C), n-butyl methacrylate(20°C), isobutyl methacrylate (53°C), sec-butyl methacrylate (60°C), tert-butyl methacrylate (118°C), cyclohexyl methacrylate (83°C), benzyl methacrylate (54°C), isobornyl methacrylate (170°C), and 2-hydroxyethyl methacrylate (75°C). One or more of these can be used. The temperature in the parenthesis is Tg of the homopolymer of each monomer.

Among these, from the viewpoint of controlling Tg of ionic polymer particles, and from the viewpoint of easiness in emulsion polymerization to produce polymer particles, and availability and economic potential, one or more selected from the group consisting of styrene, vinyl acetate, methyl methacrylate and ethyl methacrylate are preferred, one or more selected from the group consisting of styrene, methyl methacrylate and ethyl methacrylate are more preferred, and one or more selected from the group consisting of styrene and methyl methacrylate are even more preferred.

<<Monomer (a2)>>

The monomer (a2) is a (meth)acrylate such that the homopolymer thereof has a glass transition temperature of 5°C or lower, and is preferably a (meth)acrylate of the general formula (1) such that the homopolymer thereof has a glass transition temperature of 5°C or lower.
From the viewpoint of easiness in emulsion polymerization to produce polymer particles, and availability and economic potential, an alkyl (meth)acrylate is preferred among (meth)acrylates, in which the carbon number of the alkyl moiety is preferably 1 or more and 24 or less, more preferably 1 or more and 12 or less, even more preferably 1 or more and 8 or less, further more preferably 1 or more and 6 or less.
Specific examples of the monomer (a2) include ethyl acrylate (-24°C), n-butyl acrylate (-54°C), 2-ethylhexyl acrylate (-50°C), 2-ethylhexyl methacrylate (-10°C), isodecyl methacrylate (-41°C), and lauryl methacrylate (-65°C), and one or more among these can be used. The temperature in the parenthesis is Tg of the homopolymer of each monomer.
Among these, from the viewpoint of controlling Tg of ionic polymer particles and from the viewpoint of easiness in emulsion polymerization to produce polymer particles, and availability and economic potential, one or more selected from the group consisting of ethyl acrylate, n-butyl acrylate and 2-ethylhexyl acrylate are preferred, and n-butyl acrylate is more preferred.
From the viewpoint of controlling Tg of ionic polymer particles, the combination of the monomer (a1) and the monomer (a2) is preferably such that the difference between the glass transition temperature of the homopolymer of the monomer (a1) and the glass transition temperature of the homopolymer of the monomer (a2) is 15°C or more.
From the viewpoint of controlling Tg of ionic polymer particles, the glass transition temperature difference is preferably 50°C or more, more preferably 80°C or more, even more preferably 100°C or more, further more preferably 120°C or more, further more preferably 140°C or more. From the viewpoint of controlling Tg of ionic polymer particles and from the viewpoint of monomer availability and economic potential, the glass transition temperature difference is preferably 200°C or less, more preferably 180°C or less, even more preferably 170°C or less.
A specific range of the difference between the glass transition temperature of the homopolymer of the monomer (a1) and the glass transition temperature of the homopolymer of the monomer (a2) is preferably 15 to 200°C, more preferably 50 to 200°C, even more preferably 50 to 180°C, further more preferably 80 to 180°C, further more preferably 100 to 180°C, further more preferably 120 to 180°C, further more preferably 140 to 170°C.
In the case where a combination of the monomer (a1) and the monomer (a2) is used as the hydrophobic monomer (a), the ratio by mass (a1)/(a2) is preferably 50/50 to 99/1, more preferably 50/50 to 90/10, even more preferably 54/46 to 85/15, further more preferably 54/46 to 80/20, further more preferably 60/40 to 80/20, further more preferably 65/35 to 80/20. The ratio falling within the range makes it easy to control Tg of ionic polymer particles to fall within a range of higher than 5°C and 120°C or lower, and enhances the effect of stickiness suppression and the effect of wear resistance improvement.
The total content of the monomer (a1) and the monomer (a2) in the hydrophobic monomer (a) is, from the viewpoint of controlling Tg of ionic polymer particles and from the viewpoint of easiness in emulsion polymerization to produce polymer particles, preferably 50% by mass or more, more preferably 70% by mass or more, even more preferably 80% by mass or more, further more preferably 90% by mass or more. The upper limit is 100% by mass.

(Ionic Hydrophilic Monomer and Salt thereof (b))

The component (A) has a structural unit derived from an ionic hydrophilic monomer or a salt thereof (b). Having the structural unit, the component (A) can form emulsion particles dispersed in an aqueous medium.
The ionic hydrophilic monomer or a salt thereof includes an anionic group-having anionic hydrophilic monomer or a salt thereof, and a cationic group-having cationic hydrophilic monomer or a salt thereof.
The anionic group in the anionic hydrophilic monomer includes a carboxy group, a sulfonic acid group and a phosphoric acid group, and from the viewpoint of easiness in emulsion polymerization to produce polymer particles, and availability and economic potential, one or more selected from the group consisting of a carboxy group and a sulfonic acid group are preferred.
Specific examples of the anionic hydrophilic monomer or a salt thereof include a carboxy group-having vinyl compound such as (meth)acrylic acid, maleic acid, fumaric acid, itaconic acid, crotonic acid, and styrene-carboxylic acid, and a salt thereof; a sulfonic acid group-having vinyl compound such as 2-(meth)acrylamide-2-methylpropanesulfonic acid, styrenesulfonic acid, and (meth)acryloyloxyethylsulfonic acid, and a salt thereof; and a phosphoric acid group-having vinyl compound such as vinylphosphonic acid, (meth)acryloyloxyethylphosphoric acid, and a salt thereof. One or more of these can be used.
The cationic hydrophilic monomer and a salt thereof include a dialkylamino group-having (meth)acrylate or (meth)acrylamide such as dimethylaminoethyl (meth)acrylate, diethylaminoethyl (meth)acrylate, dimethylaminopropyl(meth)acrylamide, diethylaminopropyl(meth)acrylamide, and a salt or a quaternary salt thereof; and a diallylamine

compound such as diallylmethylamine, and diallylamine, and a salt or a quaternary salt thereof. One or more of these can be used.

Among the above, from the viewpoint of latitude in formulation of external preparations, and from the viewpoint of easiness in emulsion polymerization to produce polymer particles, and availability and economic potential, the ionic hydrophilic monomer or a salt thereof (b) is preferably an anionic hydrophilic monomer or a salt thereof, more preferably a monomer having one or more anionic groups such as a carboxy group and a sulfonic acid group, or a salt thereof, even more preferably one or more selected from the group consisting of acrylic acid, methacrylic acid, a sulfonic acid group-containing monomer, and salts thereof, and is, from the viewpoint of easiness in emulsion polymerization to produce polymer particles, availability and economic potential, further more preferably one or more selected from the group consisting of acrylic acid, methacrylic acid, styrenesulfonic acid, and salts thereof, further more preferably one or more selected from the group consisting of acrylic acid, methacrylic acid and salts thereof, further more preferably acrylic acid or a salt thereof.

The component (A) contains structural units derived from a hydrophobic monomer (a), and an ionic hydrophilic monomer or a salt thereof (b), and the ratio by mass of (a) to (b), (a)/(b) is 99.5/0.5 to 80/20. When the ratio by mass of (a)/(b) falls within the range, Tg of the ionic polymer particles of the component (A) can be readily controlled to fall within a desired range, and emulsion stability is good.

From the above-mentioned viewpoint, the ratio by mass (a)/(b) is preferably 99/1 to 85/15, more preferably 99/1 to 90/10, even more preferably 99/1 to 95/5, further more preferably 98.5/1.5 to 95/5, further more preferably 98/2 to 95/5.

The component (A) may further have a structural unit derived from any other monomer than the hydrophobic monomer (a) and the ionic hydrophilic monomer or a salt thereof (b), but from the viewpoint of emulsion stability, wear resistance of film, and stickiness suppression, the total content of the hydrophobic monomer (a) and the ionic hydrophilic monomer or a salt thereof (b) in all the monomers constituting the component (A) is preferably 80% by mass or more, more preferably 90% by mass or more, even more preferably 95% by mass or more, further more preferably 98% by mass or more. The upper limit is 100% by mass.

(Production Method for Component (A))

The ionic polymer particles of the component (A) can be produced by polymerizing the above-mentioned monomer component and microparticulating the resulting polymer into fine particles. The polymerizing and microparticulating method includes (1) a method of microparticulation with polymerization of a monomer component according to an emulsion polymerization method, a suspension polymerization method or a dispersion polymerization method, and (2) a method of microparticulation including polymerizing a monomer component according to a solution polymerization method to give a polymer, and then microparticulating the resultant polymer according to a phase inversion emulsification method or a suspension method. Among these, from the viewpoint of easiness in production, the method (1) is preferred, and the emulsion polymerization method is more preferred. Among the emulsion polymerization, a soap-free emulsion polymerization method with no surfactant addition is preferred, from the viewpoint of low skin irritation and good waterproofness of the resultant component (A).

The soap-free emulsion polymerization method is a method of polymerizing a monomer component in emulsion in the presence of a polymerization initiator not using an emulsifier such as a surfactant, a polymer emulsifier, or a reactive surfactant, and is a known method. In the case of emulsion polymerization, the main component of the solvent to be used is water, and as the case may be, a hydrophilic solvent such as a lower alcohol may be mixed therein. The reaction temperature is set to be not higher than the boiling point of the solvent. The monomer concentration in the reaction system is not specifically limited but is, from the viewpoint of production efficiency and coagulation inhibition, preferably 1 to 60% by mass.

According to the above-mentioned method, the component (A) can be produced in the form of an aqueous dispersion (emulsion) of ionic polymer particles. From the viewpoint of stability and handleability, preferably, the component of this form is blended in the external preparation of the present invention.

From the viewpoint of easiness in production and latitude in monomer formulation, the reaction mode is preferably radical polymerization reaction.

The radical polymerization initiator for use in the radical polymerization reaction may be any known compound, and examples thereof include a peroxide initiator such as ammonium persulfate, sodium persulfate, potassium persulfate, benzoyl peroxide, and lauroyl peroxide; and an azo-type initiator such as 2,2'-azobis(2-methylpropionamidine) dihydrochloride, 2,2'-azobis(2,4-dimethylvaleronitrile), and 2,2'-azobisisobutyronitrile. From the viewpoint of soap-free emulsion polymerization, a water-soluble radical polymerization initiator is preferred, and one or more selected from the group consisting of ammonium persulfate, sodium persulfate and potassium persulfate are more preferred.

The amount of the radical polymerization initiator to be used can be appropriately selected depending on the kind and the concentration of the monomer component, the kind of the radical polymerization initiator, and the polymerization temperature, and is, in general, preferably 0.01% by mass or more and 10% by mass or less relative to the total monomer amount, more preferably 0.1% by mass or more and 5% by mass or less.

The average particle size of the component (A) is, from the viewpoint of film wear resistance improvement and waterproofness improvement, preferably 150 nm or more, more preferably 200 nm or more, even more preferably 300 nm or more. Also from the viewpoint of emulsion stability, the average particle size of the component (A) is preferably 800 nm or less, more preferably 700 nm or less, even more preferably 600 nm or less, further more preferably 550 nm or less. Specifically, the range of the average particle size of the component (A) is preferably 150 to 800 nm, more preferably 200 to 700 nm, even more preferably 300 to 600 nm, further more preferably 300 to 550 nm.

In this description, the average particle size of the component (A) means a median diameter (D50). The average particle size is a value measured at 25°C using a laser diffraction/scattering particle size distribution measuring device, and is specifically measured according to the method described in the section of Examples.

When the component (A) is produced according to the soap-free emulsion polymerization method, the average particle size can be readily controlled to fall within the above range. In addition, the the soap-free emulsion polymerization method is preferred since use of a surfactant is unnecessary and skin irritation by the component (A) can be thereby reduced.

The content of the component (A) in the external preparation is, from the viewpoint of film wear resistance improvement and stickiness suppression, preferably 0.1% by mass or more, more preferably 0.3% by mass or more, even more preferably 0.5% by mass or more, further more preferably 0.8% by mass or more, further more preferably 1.2% by mass or more. From the viewpoint of stickiness suppression, the content is preferably 5% by mass or less, more preferably 4% by mass or less, even more preferably 3.5% by mass or less, further more preferably 3% by mass or less, further more preferably 2.5% by mass or less. Specifically, the content of the component (A) in the external preparation is preferably 0.1 to 5% by mass, more preferably 0.3 to 4% by mass, even more preferably 0.5 to 3.5 % by mass, further more preferably 0.8 to 3% by mass, further more preferably 1.2 to 2.5% by mass.

<Component (B)>

The external preparation of the present invention contains, as the component (B), one or more compounds selected from the group consisting of a phenoxyethanol (B1), a 2-ethylhexyl paramethoxycinnamate (B2), a benzyl alcohol (B3), a sorbitol (B4), an oxyalkylene derivative (B5) represented by the following general formula (I), a polyoxyalkylene alkyl glucoside (B6), an oleic acid (B7), an alkyl benzoate (B8), a xylitol (B9), a 2-ethylhexyl (RS)-2-cyano-3,3-diphenylprop-2-enoate (B10), and a methylphenylpolysiloxane (B11):

$$Z\text{-}\{O(PO)_1(EO)_m\text{-}(BO)_nH\}_a \qquad (I)$$

wherein Z represents a residue of a compound containing 3 or more and 9 or less hydroxy groups, as derived by removing a's hydroxy groups from the compound, PO represents an oxypropylene group, EO represents an oxyethylene group, BO represents an oxyalkylene group having 4 carbon atoms, a falls within a range of 3 or more and 9 or less, 1, m and n each represent an average addition molar number of PO, EO and BO units, respectively, 1 is 0.5 or more and 10 or less, m is 1 or more and 20 or less, n is 0.5 or more and 5 or less, provided that the ratio by mass (PO/EO) falls within a range of 1/5 to 5/1.

As containing the component (B), it is considered that the external preparation of the present invention can have an improved film forming performance and can better the wear resistance and the waterproofness of the film formed of the external preparation owing to the above-mentioned effects, even though Tg of the component (A) falls within a relatively high range of higher than 5°C. As the component (B), one or more of the compounds can be used.

Among the component (B), the oxyalkylene derivative (B5) represented by the following general formula (I), the polyoxyalkylene alkyl glucoside (B6), the alkyl benzoate (B8), the 2-ethylhexyl (RS)-2-cyano-3,3-diphenylprop-2-enoate (B10), and the methylphenylpolysiloxane (B11) are described.

(Oxyalkylene Derivative (B5) represented by general formula (I))

The component (B5) is an oxyalkylene derivative represented by the following general formula (I). As the component (B5), a mixture of different oxyalkylene derivatives represented by the following general formula (I) can be used.

$$Z\text{-}\{O(PO)_1(EO)_m\text{-}(BO)_nH\}_a \qquad (I)$$

wherein Z represents a residue of a compound containing 3 or more and 9 or less hydroxy groups, as derived by removing a's hydroxy groups from the compound, PO represents an oxypropylene group, EO represents an oxyethylene group,

BO represents an oxyalkylene group having 4 carbon atoms, a falls within a range of 3 or more and 9 or less, 1, m and n each represent an average addition molar number of PO, EO and BO units, respectively, 1 is 0.5 or more and 10 or less, m is 1 or more and 20 or less, n is 0.5 or more and 5 or less, provided that the ratio by mass (PO/EO) falls within a range of 1/5 to 5/1.

In the general formula (I), Z represents a residue of a compound containing 3 or more and 9 or less hydroxy groups, as derived by removing a's hydroxy groups from the compound. a falls within a range of 3 or more and 9 or less.

Regarding the compound containing 3 or more and 9 or less hydroxy groups, examples of the compound having 3 hydroxy groups include glycerin and trimethylolpropane, examples of the compound having 4 hydroxy groups include erythritol, pentaerythritol, sorbitol, alkyl glucoside and diglycerin. Examples of the compound having 5 hydroxy groups include xylitol, examples of the compound having 6 hydroxy groups include dipentaerythritol, sorbitol, and inositol, examples of the compound having 8 hydroxy groups include sucrose and trehalose, and examples of the compound having 9 hydroxy groups include maltitol.

From the viewpoint of film wear resistance improvement and stickiness suppression of the external preparation, compounds having 3 or more and 6 or less hydroxy groups are preferred among the compounds having 3 or more and 9 or less hydroxy groups, compounds having 3 or more and 4 or less hydroxy groups are more preferred, and compounds having 3 hydroxy groups are further preferred. Specifically, the compound is preferably one or more selected from the group consisting of glycerin and trimethylolpropane, more preferably glycerin.

Also preferably, in the compound having 3 or more and 9 or less hydroxy groups for the component (B5), all hydroxy groups are substituted with $-\{O(PO)_1(EO)_m-(BO)_nH\}$.

In the general formula (I), PO represents an oxypropylene group. 1 represents an average addition molar number of PO units, and is, from the viewpoint of film wear resistance improvement and stickiness suppression, preferably 0.5 or more, more preferably 2 or more. Also from the same viewpoint, 1 is preferably 10 or less, more preferably 5 or less. A specific range of 1 is preferably 0.5 to 10, more preferably 2 to 10, even more preferably 2 to 5.

In the general formula (I), EO represents an oxyethylene group, m represents an average addition molar number of EO units, and is, from the viewpoint of solubility in water, film wear resistance improvement and stickiness suppression, preferably 1 or more, more preferably 2 or more. Also from the viewpoint of film wear resistance improvement and stickiness suppression, m is preferably 20 or less, more preferably 10 or less, even more preferably 8 or less. A specific range of m is preferably 1 to 20, more preferably 2 to 10, even more preferably 2 to 8.

However, from the viewpoint of film wear resistance improvement, stickiness suppression, solubility in water and moisture retaining property, the ratio by mass (PO/EO) falls preferably within a range of 1/5 to 5/1, more preferably 1/4 to 4/1.

The order of adding PO and EO units is not specifically limited, and the units may be added in any form of random addition or block addition. From the viewpoint of film wear resistance improvement, random addition is preferred.

In the general formula (I), BO represents an oxyalkylene group having 4 carbon atoms, and examples thereof include an oxybutylene group and an oxytetramethylene group. An oxybutylene group is preferred. "Butylene group" as referred to herein includes $-CH_2-CH(C_2H_5)-$, $-CH(CH_3)-CH(CH_3)-$, and $-CH_2-C(CH_3)_2-$, and may be one or more of these. n represents an average addition molar number of BO units, and is, from the viewpoint of film wear resistance improvement and stickiness suppression, preferably 0.5 or more, more preferably 0.8 or more, even more preferably 1 or more, and from the viewpoint of moisture-retaining property, it is preferably 5 or less, more preferably 4 or less. A specific range of n is preferably 0.5 to 5, more preferably 0.8 to 4, even more preferably 1 to 4.

As in the general formula (I), the $(BO)_n$ unit bonds to the terminal hydrogen atom of the oxyalkylene derivative.

Examples of commercial products of the oxyalkylene derivative represented by the general formula (I) include "WILBRIDE S-753" (PEG/PPG/polybutylene glycol-8/5/3 glycerin) by NOF Corporation.

(Polyoxyalkylene Alkyl Glucoside (B6))

The polyoxyalkylene alkyl glucoside of the component (B6) includes a compound of an alkyl glucoside added with an alkylene oxide.

The oxyalkylene is, from the viewpoint of film wear resistance improvement and stickiness suppression, preferably an oxyalkylene having 2 or 3 carbon atoms, more preferably one or more selected from the group consisting of an oxyethylene and an oxypropylene, even more preferably an oxyethylene.

The alkyl group in the alkyl glucoside is, from the viewpoint of film wear resistance improvement and stickiness suppression, preferably an alkyl group having 1 or more and 3 or less carbon atoms, more preferably a methyl group.

The average addition molar number of the alkylene oxide is, from the viewpoint of film wear resistance improvement and stickiness suppression, preferably 5 or more, more preferably 10 or more, and is preferably 30 or less, more preferably 20 or less.

Examples of commercial products of the polyoxyalkylene alkyl glucoside include "Macbiobride MG-10E" (Methyl Gluceth-10), "Macbiobride MG-20E" (Methyl Gluceth-20), "Macbiobride MG-10P" (PPG-10 methyl glucose) and "Macbiobride MG-20P" (PPG-20 methyl glucose) by NOF Corporation.

(Alkyl Benzoate (B8))

The alkyl benzoate of the component (B8) includes, from the viewpoint of film wear resistance improvement and stickiness suppression, an alkyl ester with 12 to 15 carbon atoms of benzoic acid. Examples of commercial products of the alkyl benzoate include "Finsolv TN" (alkyl (C12-15) benzoate) by Innospec Active Chemicals LLC.

(2-Ethylhexyl (RS)-2-cyano-3,3-diphenylprop-2-enoate (B10))

The component (B10) is a compound also referred to as octocrylene, and examples of commercial products thereof include "Parsol 340" by DSM Corporation.

(Methylphenylpolysiloxane (B11))

The methylphenylpolysiloxane of the component (B11) is a compound also referred to as diphenylsiloxyphenyltrimethicone, and examples of commercial products thereof include "KF-56A" by Shin-Etsu Chemical Co., Ltd.
One or more of the compounds can be used as the component (B).
Among the above, the component (B) is, from the viewpoint of stickiness suppression, preferably one or more selected from the group consisting of a phenoxyethanol (B1), a 2-ethylhexyl paramethoxycinnamate (B2), an oxyalkylene derivative (B5) represented by the general formula (I), an alkyl benzoate (B8), a 2-ethylhexyl (RS)-2-cyano-3,3-diphenylprop-2-enoate (B10), and a methylphenylpolysiloxane (B11), more preferably one or more selected from the group consisting of a phenoxyethanol (B1), a 2-ethylhexyl paramethoxycinnamate (B2), and a 2-ethylhexyl (RS)-2-cyano-3,3-diphenylprop-2-enoate (B10), even more preferably one or more selected from the group consisting of a phenoxyethanol (B1), and a 2-ethylhexyl paramethoxycinnamate (B2).
The content of the component (B) in the external preparation is, from the viewpoint of film wear resistance improvement, preferably 0.1% by mass or more, more preferably 0.3% by mass or more, even more preferably 0.5% by mass or more, further more preferably 0.8% by mass or more, further more preferably 1.2% by mass or more. Also from the viewpoint of stickiness suppression, the content is preferably 20% by mass or less, more preferably 12% by mass or less, even more preferably 8% by mass or less, further more preferably 5% by mass or less, further more preferably 3% by mass or less. A specific range of the content of the component (B) in the external preparation is preferably 0.1 to 20% by mass, more preferably 0.3 to 12% by mass, even more preferably 0.5 to 8% by mass, further more preferably 0.5 to 5% by mass, further more preferably 0.8 to 5% by mass, further more preferably 1.2 to 3% by mass.
Regarding the content of the component (A) and the component (B) in the external preparation, preferably, the content of the component (A) is 0.1 to 5% by mass and the content of the component (B) is 0.1 to 20% by mass, more preferably, the content of the component (A) is 0.3 to 4% by mass and the content of the component (B) is 0.3 to 12% by mass, even more preferably, the content of the component (A) is 0.5 to 3.5% by mass and the content of the component (B) is 0.5 to 8% by mass, further more preferably, the content of the component (A) is 0.5 to 3.5% by mass and the content of the component (B) is 0.5 to 5% by mass, further more preferably, the content of the component (A) is 0.8 to 3% by mass and the content of the component (B) is 0.8 to 5% by mass, further more preferably, the content of the component (A) is 1.2 to 2.5% by mass and the content of the component (B) is 1.2 to 3% by mass.
The content ratio of the component (A) to the component (B) in the external preparation can be appropriately selected depending on the kind of the component (A) and the component (B), and is, from the viewpoint of stickiness suppression, preferably 5/1 to 1/5 as a ratio by mass, more preferably 4/1 to 1/4, even more preferably 2/1 to 1/2.

[Aqueous Medium]

The external preparation of the present invention can contain an aqueous medium. The aqueous medium includes water; a lower alcohol such as ethanol or isopropyl alcohol; and a low-molecular diol or triol having 6 or less carbon atoms, such as 1,3-butylene glycol, glycerin, ethylene glycol, or propylene glycol. One or more selected from the group consisting of water and a lower alcohol are preferred, one or more selected from the group consisting of water and ethanol are more preferred, and even preferably, the aqueous medium contains at least water.
The content of the aqueous medium in the external preparation can be appropriately selected depending on the formulation of the external preparation and is generally within a range of 1 to 99.8% by mass. The content of the aqueous medium in the external preparation may be a residual part except all the active ingredients in the external preparation.

[Other Components]

The external preparation of the present invention may optionally contain, in addition to the above-mentioned components, a beauty component and a medically-effective component that are used depending on the use of external preparations, as well as a component generally used in external preparations such as skin cosmetics, within a range not detracting from the object of the present invention. Examples of the components include, except the component (B), an antioxidant, a UV absorbent, a surfactant, a thickener, an oiling agent, a pH regulator, a germicide, an anti-inflammatory agent, a preservative, a colorant, a chelating agent, a moisturizer, a pearly agent, ceramides, and a fragrance.

A production method for the external preparation of the present invention is not specifically limited. For example, the component (A), the component (B) and other optional components are blended according to the method described in Examples, and mixed using a known stirring device to produce the external preparation.

Regarding the above-mentioned embodiments, the present invention further discloses the following compositions.

<1> An external preparation containing the following component (A) and component (B):

Component (A):

ionic polymer particles containing structural units derived from:

(a) one or more hydrophobic monomers selected from the group consisting of styrene and a derivative thereof, a vinyl ester, and a hydrophobic acrylic monomer, and
(b) an ionic hydrophilic monomer or a salt thereof, wherein:

the ratio by mass of (a) to (b), (a)/(b) is 99.5/0.5 to 80/20, and the ionic polymer particles have a glass transition temperature of higher than 5°C and 120°C or lower, and

Component (B):
one or more compounds selected from the group consisting of a phenoxyethanol (B1), a 2-ethylhexyl paramethoxycinnamate (B2), a benzyl alcohol (B3), a sorbitol (B4), an oxyalkylene derivative (B5) represented by the following general formula (I), a polyoxyalkylene alkyl glucoside (B6), an oleic acid (B7), an alkyl benzoate (B8), a xylitol (B9), a 2-ethylhexyl (RS)-2-cyano-3,3-diphenylprop-2-enoate (B10), and a methylphenylpolysiloxane (B11):

$$Z\text{-}\{O(PO)_1(EO)_m\text{-}(BO)_nH\}_a \qquad (I)$$

wherein Z represents a residue of a compound containing 3 or more and 9 or less hydroxy groups, as derived by removing a's hydroxy groups from the compound, PO represents an oxypropylene group, EO represents an oxyethylene group, BO represents an oxyalkylene group having 4 carbon atoms, a falls within a range of 3 or more and 9 or less, 1, m and n each represent an average addition molar number of PO, EO and BO units, respectively, 1 is 0.5 or more and 10 or less, m is 1 or more and 20 or less, n is 0.5 or more and 5 or less, provided that the ratio by mass (PO/EO) falls within a range of 1/5 to 5/1.

<2> The external preparation according to <1>, wherein the glass transition temperature (Tg) of the component (A) is preferably 10°C or higher and 105°C or lower, more preferably 10°C or higher and 100°C or lower, even more preferably 15°C or higher and 100°C or lower, further more preferably 20°C or higher and 100°C or lower, further more preferably 30°C or higher and 95°C or lower, further more preferably 30°C or higher and 70°C or lower, further more preferably 30°C or higher and 50°C or lower, further more preferably 30°C or higher and 45°C or lower.
<3> The external preparation according to <1> or <2>, wherein the hydrophobic acrylic monomer is a (meth)acrylate represented by the following general formula (1):

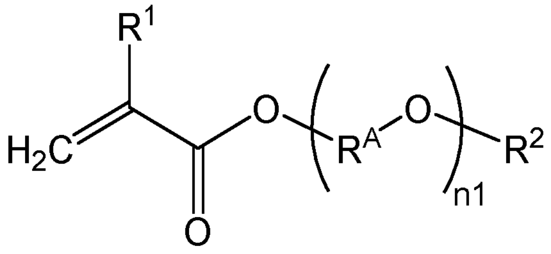

(1)

In the formula (1), $R^1$ represents a hydrogen atom or a methyl group, $R^2$ represents a chainlike aliphatic group having 1 or more and 24 or less carbon atoms, a cycloaliphatic group having 5 or more and 24 or less carbon atoms, an aryl group having 6 or more and 24 or less carbon atoms, or an aralkyl group having 7 or more and 24 or less carbon atoms, which may optionally have a hydroxy group, $R^A$ represents an alkylene group having 2 or more and 4 or less carbon atoms, and n1 represents an integer of 0 or more and 30 or less.

<4> The external preparation according to any one of <1> to <3>, wherein the ionic hydrophilic monomer or a salt thereof (b) is one or more selected from the group consisting of acrylic acid, methacrylic acid, styrenesulfonic acid and salts thereof.

<5> The external preparation according to any one of <1> to <4>, wherein the hydrophobic monomer (a) contains the following monomer (a1) and monomer (a2):

(a1) One or more selected from the group consisting of styrene, vinyl acetate, and a (meth)acrylate such that the homopolymer thereof has a glass transition temperature of higher than 5°C, and

(a2) A (meth)acrylate such that the homopolymer thereof has a glass transition temperature of 5°C or lower.

<6> The external preparation according to <5>, wherein the difference between the glass transition temperature of the homopolymer of the monomer (a1) and the glass transition temperature of the homopolymer of the monomer (a2) is preferably 15°C or more and 200°C or less, more preferably 50°C or more and 200°C or less, even more preferably 50°C or more and 180°C or less, further more preferably 80°C or more and 180°C or less, further more preferably 100°C or more and 180°C or less, further more preferably 120°C or more and 180°C or less, further more preferably 140°C or more and 170°C or less.

<7> The external preparation according to any one of <1> to <6>, wherein the average particle size of the component (A) is preferably 150 nm or more and 800 nm or less, more preferably 200 nm or more and 700 nm or less, even more preferably 300 nm or more and 600 nm or less, further more preferably 300 nm or more and 550 nm or less.

<8> The external preparation according to any one of <5> to <7>, wherein the ratio by mass of the monomer (a1) to the monomer (a2), (a1)/(a2) is preferably 50/50 to 99/1, more preferably 50/50 to 90/10, even more preferably 54/46 to 85/15, further more preferably 54/46 to 80/20, further more preferably 60/40 to 80/20, further more preferably 65/35 to 80/20.

<9> The external preparation according to any one of <1> to <8>, wherein the the total amount of the hydrophobic monomer (a) and the ionic hydrophilic monomer or a salt thereof (b) in all the monomers constituting the component (A) is preferably 80% by mass or more, more preferably 90% by mass or more, even more preferably 95% by mass or more, further more preferably 98% by mass or more.

<10> The external preparation according to any one of <5> to <9>, wherein the total amount of the monomer (a1) and the monomer (a2) in the hydrophobic monomer (a) is preferably 50% by mass or more, more preferably 70% by mass or more, even more preferably 80% by mass or more, further more preferably 90% by mass or more.

<11> The external preparation according to any one of <1> to <10>, wherein the content of the component (A) is preferably 0.1 to 5% by mass, more preferably 0.3 to 4% by mass, even more preferably 0.5 to 3.5% by mass, further more preferably 0.8 to 3% by mass, further more preferably 1.2 to 2.5% by mass.

<12> The external preparation according to any one of <1> to <11>, wherein the component (B) is preferably one or more selected from the group consisting of a phenoxyethanol (B1), a 2-ethylhexyl paramethoxycinnamate (B2), an oxyalkylene derivative (B5) represented by the general formula (I), an alkyl benzoate (B8), a 2-ethylhexyl (RS)-2-cyano-3,3-diphenylprop-2-enoate (B10), and a methylphenylpolysiloxane (B11), more preferably one or more selected from the group consisting of a phenoxyethanol (B1), a 2-ethylhexyl paramethoxycinnamate (B2), and a 2-ethylhexyl (RS)-2-cyano-3,3-diphenylprop-2-enoate (B10), even more preferably one or more selected from the group consisting of a phenoxyethanol (B1), and a 2-ethylhexyl paramethoxycinnamate (B2).

<13> The external preparation according to any one of <1> to <12>, wherein the content of the component (B) is preferably 0.1 to 20% by mass, more preferably 0.3 to 12% by mass, even more preferably 0.5 to 8% by mass, further more preferably 0.5 to 5% by mass, further more preferably 0.8 to 5% by mass, further more preferably 1.2

to 3% by mass.
<14> The external preparation according to any one of <1> to <13>, wherein the content of the component (A) and the component (B) in the external preparation is preferably such that the content of the component (A) is 0.1 to 5% by mass and the content of the component (B) is 0.1 to 20% by mass, more preferably, the content of the component (A) is 0.3 to 4% by mass and the content of the component (B) is 0.3 to 12% by mass, even more preferably, the content of the component (A) is 0.5 to 3.5% by mass and the content of the component (B) is 0.5 to 8% by mass, further more preferably, the content of the component (A) is 0.5 to 3.5% by mass and the content of the component (B) is 0.5 to 5% by mass, further more preferably, the content of the component (A) is 0.8 to 3% by mass and the content of the component (B) is 0.8 to 5% by mass, further more preferably, the content of the component (A) is 1.2 to 2.5% by mass and the content of the component (B) is 1.2 to 3% by mass.
<15> The external preparation according to any one of <1> to <14>, wherein the content ratio of the component (A) to the component (B) in the external preparation is preferably 5/1 to 1/5 as a ratio by mass, more preferably 4/1 to 1/4, even more preferably 2/1 to 1/2.
<16> An external preparation containing the following component (A) and component (B):

Component (A):

ionic polymer particles containing structural units derived from:

(a) one or more hydrophobic monomers selected from the group consisting of styrene, vinyl acetate and an alkyl (meth)acrylate, and
(b) one or more ionic hydrophilic monomers selected from the group consisting of acrylic acid and methacrylic acid, or salts thereof, wherein:

the ratio by mass of (a) to (b), (a)/(b) is 99/1 to 95/5, and the ionic polymer particles have a glass transition temperature of 10°C or higher and 105°C or lower, and

Component (B):
one or more compounds selected from the group consisting of a phenoxyethanol (B1), a 2-ethylhexyl paramethoxycinnamate (B2), a benzyl alcohol (B3), a sorbitol (B4), an oxyalkylene derivative (B5) represented by the following general formula (I), a polyoxyalkylene alkyl glucoside (B6), an oleic acid (B7), an alkyl benzoate (B8), a xylitol (B9), a 2-ethylhexyl (RS)-2-cyano-3,3-diphenylprop-2-enoate (B10), and a methylphenylpolysiloxane (B11):

$$Z\text{-}\{O(PO)_1(EO)_m\text{-}(BO)_nH\}_a \qquad (I)$$

wherein Z represents a residue of a compound containing 3 or more and 9 or less hydroxy groups, as derived by removing a's hydroxy groups from the compound, PO represents an oxypropylene group, EO represents an oxyethylene group, BO represents an oxyalkylene group having 4 carbon atoms, a falls within a range of 3 or more and 9 or less, 1, m and n each represent an average addition molar number of PO, EO and BO units, respectively, 1 is 0.5 or more and 10 or less, m is 1 or more and 20 or less, n is 0.5 or more and 5 or less, provided that the ratio by mass (PO/EO) falls within a range of 1/5 to 5/1.

<17> The external preparation according to <16>, wherein the content of the component (A) is 0.3 to 4% by mass.
<18> The external preparation according to <16> of <17>, wherein the content of the component (B) is 0.3 to 12% by mass.
<19> The external preparation according to any one of <16> to <18>, wherein the content of the component (A) is 0.3 to 4% by mass and the content of the component (B) is 0.3 to 12% by mass.
<20> The external preparation according to any one of <16> to <19>, wherein the content ratio of the component (A) to the component (B) in the external preparation is 5/1 to 1/5 as a ratio by mass.

Examples

Hereinunder the present invention is described with reference to Examples, but the present invention is not limited to the scope of Examples. In these Examples, measurement and evaluation were carried out according to the following methods.

(Glass Transition Temperature (Tg))

The glass transition temperature of polymer particles was determined according to the following mathematical expression, using the value of Tg of the homopolymer of each monomer used in Examples.

$$\frac{1}{273+Tg}=\frac{w_1}{273+Tg_1}+\frac{w_2}{273+Tg_2}+\cdot\cdot\cdot \quad (1)$$

wherein $w_1$, $w_2$, ... each are a weight fraction of each monomer, and the total of the weight fractions is 1; $Tg_1$, $Tg_2$, ... each are Tg (°C) of the homopolymer of each monomer.

Tg of the homopolymer of each monomer used in Examples is as follows.

Polymethyl methacrylate: 105°C
Polystyrene: 100°C
Polyvinyl acetate: 32°C
Poly-n-butyl acrylate: -54°C
Polyacrylic acid: 106°C

(Average Particle Size of Polymer Particles)

The average particle size (median diameter: D50) of polymer particles was determined at 25°C, using a laser diffraction/scattering particle size distribution measuring device "LA-920" from HORIBA, Ltd., and using water as a dispersion medium with a relative refractive index: 1.200 to 0.000i.

(Stickiness)

20 μl of the external preparation produced in each Example was applied to the inner side of the forearm in a circle having a diameter of 5 cm of five expert panelists, and spread on the skin at 25°C and 57% RH taking 20 seconds. Subsequently, the feeling in use (sticky feeling) was evaluated according to the following evaluation criteria, and an average of the scores of the 5 panelists was calculated.

5: Absolutely no sticky feeling at all.
4: No sticky feeling.
3: Some sticky feeling.
2: Sticky feeling.
1: Extreme sticky feeling.

(Wear Resistance)

100 μL of the external preparation produced in each Example was applied to a glass plate of 3 cm × 10 cm, and dried at 25°C for 60 minutes to form a film thereon. On the film, an artificial leather with a weight giving a pressure of 15 $gf/cm^2$ was put, and pulled at a speed of 100 mm/sec in the direction parallel to the surface of the glass plate. Those with no film breakage were evaluated as "good", and those with film breakage were evaluated as "no-good".

Production Example 1 (Production of dispersion of polymer particles 1)

510 g of ion-exchanged water was put into a 1-liter glass-made separable flask, and stirred in a nitrogen atmosphere for 30 minutes. The flask was heated up to about 70°C, and then after the system therein reached 70°C, a solution prepared by dissolving 1.5 g of ammonium persulfate in 15 g of ion-exchanged water was added thereto. Next, a monomer solution prepared by uniformly mixing 204 g of methyl methacrylate, 87 g of n-butyl acrylate and 9 g of acrylic acid was dropwise added to the system at a constant speed taking 3 hours. After the dropwise addition, this was kept at around 70°C for 1 hour, then heated up to around 75°C and kept as such for 3 hours to attain polymerization and aging. The resultant reaction solution was cooled, and then neutralized with 43.7 g of an aqueous 1 N-sodium hydroxide solution added thereto. Aggregates were removed through screen filtration (200 mesh) to give a dispersion of polymer particles 1 having a solid concentration of 35% by mass. The glass transition temperature of the polymer particles 1 was 39°C, and the average particle size thereof was 450 nm.

Production Examples 2 and 3 (Production of dispersions of polymer particles 2 and 3)

Dispersions of polymer particles 2 and 3 were produced according to the same method as in Production Example 1, except that the total amount 291 g of the methyl methacrylate and n-butyl acrylate was changed to the monomer composition (ratio by mass) shown in Table 1. The glass transition temperature and the average particle size of the polymer particles 2 and 3 are shown in Table 1.

Production Example 4 (Production of dispersion of polymer particles 6)

510 g of ion-exchanged water was put into a 1-liter glass-made separable flask, and stirred in a nitrogen atmosphere for 30 minutes. The flask was heated up to about 70°C, and then after the system therein reached 70°C, a solution prepared by dissolving 1.5 g of ammonium persulfate in 15 g of ion-exchanged water was added thereto. Next, a monomer solution prepared by uniformly mixing 285 g of styrene, 6 g of n-butyl acrylate and 9 g of acrylic acid was dropwise added to the system at a constant speed taking 3 hours. After the dropwise addition, this was kept at around 70°C for 1 hour, then heated up to around 75°C and kept as such for 3 hours to attain polymerization and aging. The resultant reaction solution was cooled, and then neutralized with 43.7 g of an aqueous 1 N-sodium hydroxide solution added thereto. Aggregates were removed through screen filtration (200 mesh) to give a dispersion of polymer particles 6 having a solid concentration of 35% by mass. The glass transition temperature and the average particle size of the polymer particles 6 are shown in Table 1.

Production Example 5 (Production of dispersion of polymer particles 7)

510 g of ion-exchanged water was put into a 1-liter glass-made separable flask, and stirred in a nitrogen atmosphere for 30 minutes. The flask was heated up to about 70°C, and then after the system therein reached 70°C, a solution prepared by dissolving 1.5 g of ammonium persulfate in 15 g of ion-exchanged water was added thereto. Next, a monomer solution prepared by uniformly mixing 225 g of vinyl acetate, 66 g of n-butyl acrylate and 9 g of acrylic acid was dropwise added to the system at a constant speed taking 3 hours. After the dropwise addition, this was kept at around 70°C for 1 hour, then heated up to around 75°C and kept as such for 3 hours to attain polymerization and aging. The resultant reaction solution was cooled, and then neutralized with 43.7 g of an aqueous 1 N-sodium hydroxide solution added thereto. Aggregates were removed through screen filtration (200 mesh) to give a dispersion of polymer particles 7 having a solid concentration of 35% by mass. The glass transition temperature and the average particle size of the polymer particles 7 are shown in Table 1.

Production Example 6 (Production of dispersion of polymer particles 8)

A dispersion of polymer particles 8 was produced according to the same method as in Production Example 4, except that the total amount 291 g of styrene and n-butyl acrylate was changed to the monomer composition (ratio by mass) shown in Table 1. The glass transition temperature and the average particle size of the polymer particles 8 are shown in Table 1.

Comparative Production Examples 1 and 2 (Production of dispersions of polymer particles 4 and 5)

Dispersions of comparative polymer particles 4 and 5 were produced according to the same method as in Production Example 1, except that the total amount 291 g of methyl methacrylate and n-butyl acrylate was changed to the monomer composition (ratio by mass) shown in Table 1. The glass transition temperature and the average particle size of the polymer particles are shown in Table 1.

Table 1

| | | Monomer Composition (ratio by mass) | | | | | Ratio by mass (a1)/(a2) | Properties | |
|---|---|---|---|---|---|---|---|---|---|
| | | Methyl Methacrylate (a1) | Styrene (a1) | Vinyl Acetate (a1) | n-Butyl Acrylate (a2) | Acrylic Acid (b) | | Glass Transition Temperature (°C) | Average Particle Size (nm) |
| Production Example 1 | Polymer Particles 1 | 68 | - | - | 29 | 3 | 70/30 | 39 | 450 |
| Production Example 2 | Polymer Particles 2 | 60 | - | - | 37 | 3 | 62/38 | 25 | 460 |
| Production Example 3 | Polymer Particles 3 | 54 | - | - | 43 | 3 | 56/44 | 15 | 390 |
| Production Example 4 | Polymer Particles 6 | - | 95 | - | 2 | 3 | 98/2 | 95 | 150 |
| Production Example 5 | Polymer Particles 7 | - | - | 75 | 22 | 3 | 77/23 | 9 | 150 |
| Production Example 6 | Polymer Particles 8 | - | 68 | - | 29 | 3 | 70/30 | 37 | 160 |
| Comparative Production Example 1 | Polymer Particles 4 | 16 | - | - | 81 | 3 | 16/84 | -35 | 310 |
| Comparative Production Example 2 | Polymer Particles 5 | 47 | - | - | 50 | 3 | 48/52 | 4 | 360 |

Examples 1 to 25, Comparative Examples 1 to 18 (Production and evaluation of external preparations)

The components shown in Table 2 were mixed to produce oil-in-water type external preparations of Examples and Comparative Examples. The blending amount shown in Table 2 is an effective ingredient amount (% by mass) of each component. The resultant external preparations were tested to evaluate the stickiness and the wear resistance thereof according to the above-mentioned methods. The results are shown in Table 2.

The preparations of Comparative Examples 5 to 18 were poor in wear resistance, and were therefore not tested for stickiness evaluation.

Table 2

| | Polymer Particles | | | Component (B) or Component (B') | | | Other Compound | | Water | Evaluation | |
|---|---|---|---|---|---|---|---|---|---|---|---|
| | Kind | Glass Transition Temperature (°C) | Content (% by mass) | Kind | | Content (% by mass) | Kind | Content (% by mass) | Content | Stickiness | Wear Resistance |
| Example 1 | Polymer Particles 1 | 39 | 2 | B1 | phenoxyethanol | 2 | - | - | balance | 4.6 | good |
| Example 2 | Polymer Particles 1 | 39 | 2 | B1 | phenoxyethanol | 0.5 | - | - | balance | 4.8 | good |
| Example 3 | Polymer Particles 1 | 39 | 0.5 | B1 | phenoxyethanol | 0.5 | - | - | balance | 4.6 | good |
| Example 4 | Polymer Particles 1 | 39 | 2 | B2 | 2-ethylhexyl paramethoxycinnamate *1 | 2 | - | - | balance | 4.6 | good |
| Example 5 | Polymer Particles 1 | 39 | 2 | B2 | 2-ethylhexyl paramethoxycinnamate *1 | 2 | ethanol | 20 | balance | 4.8 | good |
| Example 6 | Polymer Particles 1 | 39 | 2 | B2 | 2-ethylhexyl paramethoxycinnamate *1 | 8 | ethanol | 20 | balance | 4.0 | good |
| Example 7 | Polymer Particles 1 | 39 | 2 | B2 | 2-ethylhexyl paramethoxycinnamate *1 | 0.5 | - | - | balance | 4.8 | good |
| Example 8 | Polymer Particles 1 | 39 | 0.5 | B2 | 2-ethylhexyl paramethoxycinnamate *1 | 0.5 | - | - | balance | 4.6 | good |
| Example 9 | Polymer Particles 1 | 39 | 2 | B3 | benzyl alcohol | 2 | - | - | balance | 4.2 | good |
| Example 10 | Polymer Particles 1 | 39 | 2 | B4 | sorbitol | 2 | - | - | balance | 4.0 | good |
| Example 11 | Polymer Particles 1 | 39 | 2 | B5 | oxyalkylene derivative represented by the formula (I)*2 | 2 | - | - | balance | 4.4 | good |

(continued)

| | Polymer Particles | | | Component (B) or Component (B') | | | Other Compound | | Water | Evaluation | |
|---|---|---|---|---|---|---|---|---|---|---|---|
| | Kind | Glass Transition Temperature (°C) | Content (% by mass) | Kind | | Content (% by mass) | Kind | Content (% by mass) | Content | Stickiness | Wear Resistance |
| Example 12 | Polymer Particles 1 | 39 | 2 | B6 | polyoxyalkylene alkyl glucoside*3 | 2 | - | - | balance | 3.8 | good |
| Example 13 | Polymer Particles 1 | 39 | 2 | B7 | oleic acid | 2 | - | - | balance | 3.4 | good |
| Example 14 | Polymer Particles 1 | 39 | 2 | B8 | alkyl(C12-15) benzoate*4 | 2 | ethanol | 20 | balance | 4.4 | good |
| Example 15 | Polymer Particles 1 | 39 | 2 | B9 | xylitol | 2 | - | - | balance | 4.0 | good |
| Example 16 | Polymer Particles 1 | 39 | 2 | B10 | octocrylene*5 | 2 | - | - | balance | 4.6 | good |
| Example 17 | Polymer Particles 1 | 39 | 2 | B11 | methylphenylpolysiloxane*6 | 2 | - | - | balance | 4.4 | good |
| Example 18 | Polymer Particles 2 | 25 | 2 | B1 | phenoxyethanol | 2 | - | - | balance | 3.8 | good |
| Example 19 | Polymer Particles 3 | 15 | 2 | B1 | phenoxyethanol | 2 | - | - | balance | 3.2 | good |
| Example 20 | Polymer Particles 6 | 95 | 2 | B1 | phenoxyethanol | 2 | - | - | balance | 4.6 | good |
| Example 21 | Polymer Particles 6 | 95 | 2 | B2 | 2-ethylhexyl paramethoxycinnamate *1 | 2 | ethanol | 20 | balance | 4.4 | good |
| Example 22 | Polymer Particles 7 | 9 | 2 | B1 | phenoxyethanol | 2 | - | - | balance | 3.4 | good |
| Example 23 | Polymer Particles 7 | 9 | 2 | B2 | 2-ethylhexyl paramethoxycinnamate *1 | 2 | ethanol | 20 | balance | 3.2 | good |
| Example 24 | Polymer Particles 8 | 37 | 2 | B1 | phenoxyethanol | 2 | - | - | balance | 4.4 | good |

(continued)

| | Polymer Particles | | | Component (B) or Component (B') | | | Other Compound | | Water | Evaluation | |
|---|---|---|---|---|---|---|---|---|---|---|---|
| | Kind | Glass Transition Temperature (°C) | Content (% by mass) | Kind | | Content (% by mass) | Kind | Content (% by mass) | Content | Stickiness | Wear Resistance |
| Example 25 | Polymer Particles 8 | 37 | 2 | B2 | 2-ethylhexyl paramethoxycinnamate *1 | 2 | ethanol | 20 | balance | 4.0 | good |
| Comparative Example 1 | Polymer Particles 1 | 39 | 2 | - | - | - | - | - | balance | 5.0 | no-good |
| Comparative Example 2 | Polymer Particles 3 | 15 | 2 | - | - | - | - | - | balance | 4.2 | no-good |
| Comparative Example 3 | Polymer Particles 4 | -35 | 2 | B1 | phenoxyethanol | 2 | - | - | balance | 1.4 | good |
| Comparative Example 4 | Polymer Particles 5 | 4 | 2 | B1 | phenoxyethanol | 2 | - | - | balance | 2.2 | good |
| Comparative Example 5 | Polymer Particles 1 | 39 | 2 | B1' | propylene glycol | 2 | - | - | balance | - | no-good |
| Comparative Example 6 | Polymer Particles 1 | 39 | 2 | B2' | glycerin | 2 | - | - | balance | - | no-good |
| Comparative Example 7 | Polymer Particles 1 | 39 | 2 | B3' | propanediol | 2 | - | - | balance | - | no-good |
| Comparative Example 8 | Polymer Particles 1 | 39 | 2 | B4' | 1,3-butanediol | 2 | - | - | balance | - | no-good |
| Comparative Example 9 | Polymer Particles 1 | 39 | 2 | B5' | polyethylene glycol*7 | 2 | - | - | balance | - | no-good |
| Comparative Example 10 | Polymer Particles 1 | 39 | 2 | B6' | dimethylpolysiloxane*8 | 2 | - | - | balance | - | no-good |
| Comparative Example 11 | Polymer Particles 1 | 39 | 2 | B7' | hydrogenated polyisobutene*9 | 2 | - | - | balance | - | no-good |
| Comparative Example 12 | Polymer Particles 1 | 39 | 2 | B8' | olive oil*10 | 2 | - | - | balance | - | no-good |

(continued)

| | Polymer Particles | | | Component (B) or Component (B') | | | Other Compound | | Water | Evaluation | |
|---|---|---|---|---|---|---|---|---|---|---|---|
| | Kind | Glass Transition Temperature (°C) | Content (% by mass) | Kind | | Content (% by mass) | Kind | Content (% by mass) | Content | Stickiness | Wear Resistance |
| Comparative Example 13 | Polymer Particles 1 | 39 | 2 | B9' | isopropyl palmitate*11 | 2 | - | - | balance | - | no-good |
| Comparative Example 14 | Polymer Particles 1 | 39 | 2 | B10' | neopentyl glycol dicaprylate*12 | 2 | - | - | balance | - | no-good |
| Comparative Example 15 | Polymer Particles 1 | 39 | 2 | B11' | isononyl isononanoate*13 | 2 | - | - | balance | - | no-good |
| Comparative Example 16 | Polymer Particles 1 | 39 | 2 | B12' | isopropyl myristate*14 | 2 | - | - | balance | - | no-good |
| Comparative Example 17 | Polymer Particles 1 | 39 | 2 | B13' | caprylylmethicone*15 | 2 | - | - | balance | - | no-good |
| Comparative Example 18 | Polymer Particles 1 | 39 | 2 | B14' | triethylhexanoin*16 | 2 | - | - | balance | - | no-good |

The formulation ingredients in Table 2 are as follows.

*1 2-Ethylhexyl paramethoxycinnamate: Uvinul MC80 (by BASF SE)
*2 Oxyalkylene derivative represented by the formula (I) (PEG/PPG/polybutylene glycol-8/5/3 glycerin): WILBRIDE S-753 (by NOF Corporation)
*3 Polyoxyalkylene alkyl glucoside (Methyl Gluceth-20): Macbiobride MG-20E (by NOF Corporation)
*4 Alkyl(C12-15) benzoate: Finsolv TN (by Innospec Active Chemicals LLC)
*5 Octocrylene: Parsol 340 (by DSM Corporation)
*6 Methylphenylpolysiloxane: KF-56A (by Shin-Etsu Chemical Co., Ltd.)
*7 Polyethylene glycol: PEG-1540 (by Sanyo Chemical Industries, Ltd.)
*8 Dimethylpolysiloxane: Silicone KF-96L-2CS (by Shin-Etsu Chemical Co., Ltd.)
*9 Hydrogenated polyisobutene: Parleam 4 (by NOF Corporation)
*10 Olive oil: Cropure OL (by Crode Japan KK)
*11 Isopropyl palmitate: Exceparl IPP (by Kao Corporation)
*12 neopentyl glycol dicaprylate: Estemol N-01 (by The Nisshin OilliO Group, Ltd.)
*13 Isononyl isononanoate: Salacos 99 (by The Nisshin OilliO Group, Ltd.)
*14 Isopropyl myristate: Exceparl IPM (by Kao Corporation)
*15 Caprylylmethicone: SS-3408 (by DuPont Toray Specialty Materials K.K.)
*16 Triethylhexanoin: T.I.O. (by The Nisshin OilliO Group, Ltd.)

From Table 2, it is known that the external preparation of the present invention that contains predetermined component (A) and component (B) has good wear resistance and is excellent in film forming performance, and the film formed of it is less sticky.

Industrial Applicability

The external preparation of the present invention is less sticky and excellent in good feeling in use, and in addition, has a high-level film forming performance and is excellent in wear resistance, and accordingly, the external preparation is useful, for example, for skin cosmetics.

## Claims

1. An external preparation comprising the following component (A) and component (B):

Component (A):

ionic polymer particles containing structural units derived from:

(a) one or more hydrophobic monomers selected from the group consisting of styrene and a derivative thereof, a vinyl ester, and a hydrophobic acrylic monomer, and
(b) an ionic hydrophilic monomer or a salt thereof, wherein:

the ratio by mass of (a) to (b), (a)/(b) is 99.5/0.5 to 80/20, and the ionic polymer particles have a glass transition temperature of higher than 5°C and 120°C or lower, and

Component (B):
one or more compounds selected from the group consisting of a phenoxyethanol (B1), a 2-ethylhexyl paramethoxycinnamate (B2), a benzyl alcohol (B3), a sorbitol (B4), an oxyalkylene derivative (B5) represented by the following general formula (I), a polyoxyalkylene alkyl glucoside (B6), an oleic acid (B7), an alkyl benzoate (B8), a xylitol (B9), a 2-ethylhexyl (RS)-2-cyano-3,3-diphenylprop-2-enoate (B10), and a methylphenylpolysiloxane (B11):

$$Z\text{-}\{O(PO)_1(EO)_m\text{-}(BO)_nH\}_a \qquad (I)$$

wherein Z represents a residue of a compound containing 3 or more and 9 or less hydroxy groups, as derived by removing a's hydroxy groups from the compound, PO represents an oxypropylene group, EO represents an oxyethylene group, BO represents an oxyalkylene group having 4 carbon atoms, a falls within a range of 3 or

more and 9 or less, 1, m and n each represent an average addition molar number of PO, EO and BO units, respectively, 1 is 0.5 or more and 10 or less, m is 1 or more and 20 or less, n is 0.5 or more and 5 or less, provided that the ratio by mass (PO/EO) falls within a range of 1/5 to 5/1.

**2.** The external preparation according to claim 1, wherein the hydrophobic acrylic monomer is a (meth)acrylate represented by the following general formula (1):

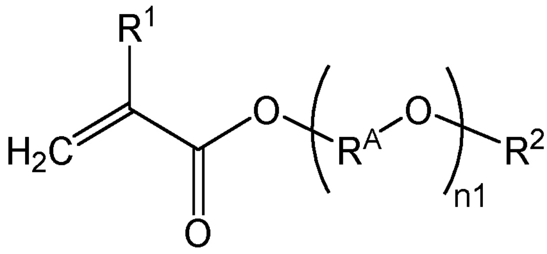

(1)

wherein $R^1$ represents a hydrogen atom or a methyl group, $R^2$ represents a chainlike aliphatic group having 1 or more and 24 or less carbon atoms, a cycloaliphatic group having 5 or more and 24 or less carbon atoms, an aryl group having 6 or more and 24 or less carbon atoms, or an aralkyl group having 7 or more and 24 or less carbon atoms, which may optionally have a hydroxy group, $R^A$ represents an alkylene group having 2 or more and 4 or less carbon atoms, and n1 represents an integer of 0 or more and 30 or less.

**3.** The external preparation according to claim 1 or 2, wherein the ionic hydrophilic monomer or a salt thereof (b) is one or more selected from the group consisting of acrylic acid, methacrylic acid, styrenesulfonic acid and salts thereof.

**4.** The external preparation according to any one of claims 1 to 3, wherein the hydrophobic monomer (a) contains the following monomer (a1) and monomer (a2):

(a1) one or more selected from the group consisting of styrene, vinyl acetate, and a (meth)acrylate such that the homopolymer thereof has a glass transition temperature of higher than 5°C, and
(a2) a (meth)acrylate such that the homopolymer thereof has a glass transition temperature of 5°C or lower.

**5.** The external preparation according to claim 4, wherein the difference between the glass transition temperature of the homopolymer of the monomer (a1) and the glass transition temperature of the homopolymer of the monomer (a2) is 15°C or more.

**6.** The external preparation according to any one of claims 1 to 5, wherein the average particle size of the component (A) is 150 nm or more and 800 nm or less.

**7.** The external preparation according to any one of claims 4 to 6, wherein the ratio by mass of the monomer (a1) to the monomer (a2), (a1)/(a2) is 50/50 to 99/1.

**8.** The external preparation according to any one of claims 1 to 7, wherein the the total amount of the hydrophobic monomer (a) and the ionic hydrophilic monomer or a salt thereof (b) in all the monomers constituting the component (A) is 80% by mass or more.

**9.** The external preparation according to any one of claims 4 to 8, wherein the total amount of the monomer (a1) and the monomer (a2) in the hydrophobic monomer (a) is 80% by mass or more.

**10.** The external preparation according to any one of claims 1 to 9, wherein the content of the component (A) is 0.1% by mass or more and 5% by mass or less, and the content of the component (B) is 0.1% by mass or more and 20% by mass or less.

**11.** The external preparation according to any one of claims 1 to 10, wherein the content ratio of the component (A) to the component (B) is 5/1 to 1/5 as a ratio by mass.

## INTERNATIONAL SEARCH REPORT

International application No.
PCT/JP2019/038448

A. CLASSIFICATION OF SUBJECT MATTER
Int.Cl. A61K8/34(2006.01)i, A61K8/36(2006.01)i, A61K8/37(2006.01)i, A61K8/81(2006.01)i, A61K8/86(2006.01)i, A61K8/891(2006.01)i, A61Q1/00(2006.01)i
According to International Patent Classification (IPC) or to both national classification and IPC

B. FIELDS SEARCHED
Minimum documentation searched (classification system followed by classification symbols)
Int.Cl. A61K8/34, A61K8/36, A61K8/37, A61K8/81, A61K8/86, A61K8/891, A61Q1/00

Documentation searched other than minimum documentation to the extent that such documents are included in the fields searched

| | |
|---|---|
| Published examined utility model applications of Japan | 1922-1996 |
| Published unexamined utility model applications of Japan | 1971-2019 |
| Registered utility model specifications of Japan | 1996-2019 |
| Published registered utility model applications of Japan | 1994-2019 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X | JP 09-125091 A (KAO CORP.) 13 May 1997, paragraphs [0001], [0024], [0037]-[0041], [0047]-[0048], [0051]-[0052] (Family: none) | 1-11 |
| X | JP 10-067617 A (BASF AG.) 10 March 1998, paragraphs [0001], [0045]-[0046], [0050] & US 6132705 A, column 1, lines 3-8, column 6, lines 27-67, table 1, example 26 & DE 19627204 A1 | 1-11 |

☒ Further documents are listed in the continuation of Box C.   ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 19 December 2019 (19.12.2019) | 07 January 2019 (07.01.2019) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No.
PCT/JP2019/038448

C (Continuation). DOCUMENTS CONSIDERED TO BE RELEVANT

| Category* | Citation of document, with indication, where appropriate, of the relevant passages | Relevant to claim No. |
|---|---|---|
| A | JP 2005-002207 A (NIPPON NSC LTD.) 06 January 2005, entire text & EP 1486514 A1 | 1-11 |
| A | JP 2011-037727 A (KAO CORP.) 24 February 2011, entire text (Family: none) | 1-11 |
| A | JP 02-262512 A (SHIONOGI & CO., LTD.) 25 October 1990, entire text & US 5346977 A, entire text & EP 391274 A2 | 1-11 |
| A | US 5614049 A (WACKER CHEMIE GMBH) 25 March 1997, entire text & WO 1995/013418 A1 & EP 728235 A1 | 1-11 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2002327019 A
- JP 2005002207 A
- JP 2006008585 A
- JP 2006008561 A


### Non-patent literature cited in the description


- **J. BRANDRUP.** Polymer Handbook. John Wiley & Sons. Inc,